(19) Europäisches Patentamt
European Patent Office
Office européen des brevets

(11) EP 2 620 148 A1

(12) EUROPEAN PATENT APPLICATION

(43) Date of publication:
**31.07.2013 Bulletin 2013/31**

(51) Int Cl.:
***A61K 31/337*** *(2006.01)* ***A61K 33/24*** *(2006.01)*
***A61P 35/00*** *(2006.01)*

(21) Application number: **12305109.6**

(22) Date of filing: **27.01.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Aventis Pharma S.A.**
**92160 Antony (FR)**

(72) Inventor: **The designation of the inventor has not yet been filed**

(74) Representative: **Gaslonde, Aude et al**
**Sanofi**
**Département Brevets**
**54, rue La Boétie**
**75008 Paris (FR)**

# (54) Antitumoral combination comprising cabazitaxel and cisplatin

(57) The present invention relates to a combination comprising cabazitaxel and cisplatin. The present invention relates also to a pharmaceutical composition containing such a combination and to a pharmaceutical kit comprising: (i) a first galenic formulation comprising cabazitaxel; and (ii) a second galenic formulation comprising cisplatin. The invention relates also to the use of this combination and/or pharmaceutical composition and/or pharmaceutical kit in the treatment of neoplastic diseases, more particularly in the treatment of cancer.



Printed by Jouve, 75001 PARIS (FR)

## Description

The present invention relates to an antitumoral combination comprising cabazitaxel, which may be in the form of anhydrous base, a hydrate or a solvate, and cisplatin. The present invention relates also to a pharmaceutical composition containing such a combination and to the use of this combination and/or pharmaceutical composition in the treatment of neoplastic diseases, more particularly in the treatment of cancer.

[State of the art]

WO96/30355 discloses taxoids derivatives, among which cabazitaxel, useful as antitumoral agents. This document discloses also a long list of other drugs that may be used as a co-treatment with such taxoids. In this list, platinum complexes such as cisplatin are cited. No data supporting such co-treatment are disclosed in this document.
W02010/128258 discloses an antitumoral combination comprising cabazitaxel and capecitabine in the treatment of metastatic breast cancer for patients progressing after a previous treatment by anthracyclines and taxanes.
A phase I study is currently on going to evaluate the effects of combining cabazitaxel with cisplatin given every 3 weeks in patients with advanced solid cancer. The disclosure published on the clinicaltrials.gov site does not give any results for this study.

[Technical problem]

There is always a need to find new antitumoral treatments.
The invention answer to that need by providing an antitumoral combination comprising cabazitaxel, which may be in the form of anhydrous base, a hydrate or a solvate, and cisplatin. In fact, it has now been demonstrated that the efficacy of cabazitaxel may be considerably improved when it is administered in combination with cisplatin. Furthermore, it has been shown that the combination according to the invention is well tolerated, does not exacerbate the toxicity of each of the antitumoral agents and allows the treatment of tumors either by stabilizing or by inducing a partial or a complete regression of the tumor.

[Brief description of the invention]

The present invention relates to an antitumoral combination comprising cabazitaxel, which may be in the form of anhydrous base, a hydrate or a solvate, and cisplatin.
The combination according to the invention is well tolerated, does not exacerbate the toxicity of each of the antitumoral agents and allows the treatment of tumors either by stabilizing or by inducing a partial or a complete regression of the tumor.
The present invention relates also to a pharmaceutical composition containing such a combination.
The present invention relates also to a pharmaceutical kit which comprises:

(i) a first galenic formulation comprising cabazitaxel in the form of a free base or of an addition salt with a pharmaceutical acceptable acid, or in the form of a hydrate or of a solvate;
(ii) a second galenic formulation comprising cisplatin;

both galenic formulations (i) et (ii) being intended to be independently administered, each administration with regard to the other one being simultaneous, separated or spread in the time.
The present invention relates also to the use of this combination and/or pharmaceutical composition and/or pharmaceutical kit in the treatment of neoplastic diseases, more particularly in the treatment of cancer..

[Definitions]

Cabazitaxel is an antitumoral agent of the taxoid family and has the following formula:

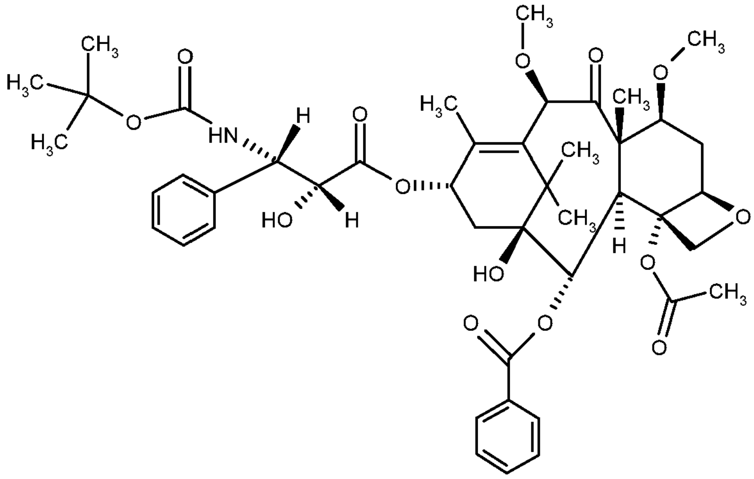

It may be in the form of anhydrous base, a hydrate or a solvate.

The chemical name of cabazitaxel is 4α-acetoxy-2α-benzoyloxy-5β,20-epoxy-1β-hydroxy-7β,10β-dimethoxy-9-oxo-11-taxen-13-yl (2R,3S)-3-*tert*-butoxycarbonylamino-2-hydroxy-3-phenylpropionate. Cabazitaxel is synonymously known as (2α,5β,7β,10β,13α)-4-acetoxy-13-({(2R,3S)-3-[(tertbutoxycarbonyl)amino]-2-hydroxy-3-phenylpropanoyl} oxy)-1-hydroxy-7,10-dimethoxy-9-oxo-5,20-epoxytax-11-en-2-yl benzoate.

This compound and a preparative method thereof is described in WO96/30355, EP0817779B1 and US5847170.

Cabazitaxel may be administered in base form (cf. above formula), or in the form of a hydrate. It may also be a solvate, i.e. a molecular complex characterized by the incorporation of the crystallization solvent into the crystal of the molecule of the active principle (see in this respect page 1276 of J. Pharm. Sci. 1975, 64(8), 1269-1288).

In particular, it may be an acetone solvate, and, more particularly, may be the solvate described in W02005/02846. It may be an acetone solvate of cabazitaxel containing between 5% and 8% and preferably between 5% and 7% by weight of acetone (% means content of acetone/content of acetone+cabazitaxel × 100). An average value of the acetone content is 7%, which approximately represents the acetone stoichiometry, which is 6.5% for a solvate containing one molecule of acetone. The procedure described below allows the preparation of an acetone solvate of cabazitaxel: 940 ml of purified water are added at 20±5°C (room temperature) to a solution of 207 g of 4α-acetoxy-2α-benzoyloxy-5β,20-epoxy-1β-hydroxy-7β,10β-dimethoxy-9-oxo-11-taxen-13α-yl (2R,3S)-3-*tert*-butoxycarbonylamino-2-hydroxy-3-phenylpropionate at about 92% by weight in about 2 litres of acetone, followed by seeding with a suspension of 2 g of 4α-acetoxy-2α-benzoyloxy-5β,20-epoxy-1β-hydroxy-7β,10β-dimethoxy-9-oxo-11-taxen-13α-yl (2R, 3S)-3-*tert*-butoxycarbonylamino-2-hydroxy-3-phenylpropionate isolated from acetone/water in a mixture of 20 ml of water and 20 ml of acetone. The resulting mixture is stirred for about 10 to 22 hours, and 1.5 litres of purified water are added over 4 to 5 hours. This mixture is stirred for 60 to 90 minutes, and the suspension is then filtered under reduced pressure. The cake is washed on the filter with a solution prepared from 450 ml of acetone and 550 ml of purified water, and then oven-dried at 55°C under reduced pressure (0.7 kPa) for 4 hours. 197 g of 4α-acetoxy-2α-benzoyloxy-5β,20-epoxy-1β-hydroxy-7β,10β-dimethoxy-9-oxo-11-taxen-13α-yl (2R,3S)-3-*tert*-butoxycarbonylamino-2-hydroxy-3-phenylpropionate acetone containing 0.1% water and 7.2% acetone (theoretical amount: 6.5% for a stoichiometric solvate) are obtained.

Cabazitaxel may be administered parenterally, such as via intravenous administration. A galenical form of cabazitaxel suitable for administration by intravenous infusion is that in which the cabazitaxel is dissolved in water in the presence of excipients chosen from surfactants, cosolvents, glucose or sodium chloride, etc. For example, a galenical form of cabazitaxel may be prepared by diluting a premix solution of cabazitaxel contained in a sterile vial (80 mg of cabazitaxel + 2 ml of solvent + Polysorbate 80) with a sterile vial containing a solution of 6 ml of water and ethanol (13% by weight of 95% ethanol) in order to obtain 8 ml of a solution ready to be rediluted in a perfusion bag. The concentration of cabazitaxel in this ready-to-redilute solution is about 10 mg/ml. The perfusion is then prepared by injecting the appropriate amount of this ready-to-redilute solution into the perfusion bag containing water and glucose (about 5%) or sodium chloride (about 0.9%). <u>Cisplatin</u> is a platinum derivative used to treat various types of cancers, including sarcomas, some carcinomas (e.g. small cell lung cancer, and ovarian cancer), lymphomas, and germ cell tumors. It was the first member of a class of anti-cancer drugs which now also includes carboplatin and oxaliplatin. These platinum complexes react in vivo, binding to and causing crosslinking of DNA which ultimately triggers apoptosis (programmed cell death).

<u>Effective quantity:</u> quantity of a pharmaceutical compound producing an effect on the treated tumor.

<u>Pharmaceutically acceptable acid:</u> organic or inorganic acid having a low toxicity (see "Pharmaceutical salts" J.Pharm.Sci. 1977, 66, 1-19);

Therapeutic synergy

The improved efficacy of the combination according to the invention may be demonstrated by the determination of the therapeutic synergy.
A combination manifests therapeutic synergy if it is therapeutically superior to the best agent of the study used alone at its maximum tolerated dose (HNTD or Highest Non Toxic Dose) or at its highest dose tested when toxicity cannot be reached in the animal species.
This efficacy may be quantified, for example, by the $\log_{10}$ cell kill, which is determined according to the following formula:

$$\log_{10} \text{ cell kill} = \text{T-C (days)}/3.32 \times T_d$$

in which T - C represents the tumor growth delay, which is the median time in days for the tumors of the treated group (T) and the tumors of the control group (C) to have reached a predetermined value (1 g for example), and $T_d$ represents the time in days needed for the volume of the tumor to double in the control animals [T.H. Corbett et al., Cancer, 40: 2660-2680 (1977); F.M. Schabel et al., Cancer Drug Development, Part B, Methods in Cancer Research, 17: 3-51, New York, Academic Press Inc. (1979)].
A product is considered to be active if $\log_{10}$ cell kill is greater than or equal to 0.7. A product is considered to be very active if $\log_{10}$ cell kill is greater than or equal to 2.8.
The combination will manifest therapeutic synergy when the $\log_{10}$ cell kill is greater than the value of the $\log_{10}$ cell kill of the best constituent administered alone at its maximum tolerated dose (by at least 1 log cell kill).
The efficacy of the combinations on solid tumors may be determined experimentally in the following manner:

> The animals subjected to the experiment, generally mice, are subcutaneously grafted bilaterally with 30 to 60 mg of a tumor fragment on day 0. The animals are implanted with a murine tumor grafted in the syngenic strain of mice of origin of the tumor, or by a rodent or human tumor xenografted in immunocompromized mice. Some days post tumor implantation, mice are randomized according to their body weight to the different groups of treatments and controls. The animals are observed every day. The different animal groups are weighed daily during treatment until the maximum weight loss is reached and subsequent full weight recovery has occurred. The groups are then weighed once or twice a week until the end of the trial.

The tumors are measured 1 to 5 times a week, depending on the tumor doubling time, until the tumor reaches approximately 2 g, or until the animal dies (if this occurs before the tumor reaches 2 g). The animals are necropsied immediately after euthanasia or death. The antitumor activity is determined in accordance with the different parameters recorded.

[Description of the invention]

The present invention relates to an antitumoral combination comprising cabazitaxel, which may be in the form of anhydrous base, a hydrate or a solvate, and cisplatin.
For example, cabazitaxel may be in the form of an acetone solvate, and, more particularly, may be the solvate described in W02005/02846.
The acetone solvate of cabazitaxel may contain between 5% and 8% and preferably between 5% and 7% by weight of acetone (% means content of acetone/content of acetone+cabazitaxel $\times$ 100). An average value of the acetone content is 7%, which approximately represents the acetone stoichiometry, which is 6.5% for a solvate containing one molecule of acetone.
The present invention also relates to an antitumoral combination comprising an effective quantity of cabazitaxel and an effective quantity of cisplatin.
The present invention also relates to an antitumoral combination which shows therapeutic synergy.
The combination according to the invention is well tolerated, does not exacerbate the toxicity of each of the antitumoral agents and allows the treatment of tumors either by stabilizing or by inducing a partial or a complete regression of the tumor.
Cabazitaxel may be administered by perfusion (intravenous infusion) at a dose from 15 to 25 mg/m$^2$, for example chosen from the following doses: 15; 20 and 25 mg/m$^2$.
Cisplatin may be administered by perfusion (intravenous infusion) at a dose of 75mg/m2.
The invention thus also concerns a combination comprising cabazitaxel and cisplatin, cabazitaxel being in the

form of a free base or of an addition salt with a pharmaceutical acceptable acid, or in the form of a hydrate or of a solvate, the combination being adapted for an administration of cabazitaxel by perfusion at a dose from 15 to 25 $mg/m^2$.

The invention thus also concerns a combination comprising cabazitaxel and cisplatin, cabazitaxel being in the form of a free base or of an addition salt with a pharmaceutical acceptable acid, or in the form of a hydrate or of a solvate, the combination being adapted for an administration of cisplatin by perfusion at a dose of 75mg/m2.

The invention thus also concerns a combination comprising cabazitaxel and cisplatin, cabazitaxel being in the form of a free base or of an addition salt with a pharmaceutical acceptable acid, or in the form of a hydrate or of a solvate, the combination being adapted for an administration of cabazitaxel by perfusion at a dose of 15 $mg/m^2$ and for an administration of cisplatin by perfusion at a dose of 75mg/m2.

The cycle of administration of the two antitumoral agents is repeated with an interval between two administrations of cabazitaxel of three weeks.

The use of cabazitaxel and cisplatin for the preparation of a combination according to the invention is also part of the invention.

The present invention relates also to the combination according to the present invention for its use as a medicament in the treatment of neoplastic diseases, more particularly in the treatment of cancer.

The present invention also relates to a pharmaceutical composition containing the combination according to the invention.

The present invention relates also to the pharmaceutical composition according to the present invention for its use as a medicament in the treatment of neoplastic diseases, more particularly in the treatment of cancer.

The invention also concerns a method of treating cancers in a patient in need thereof, said method comprising administrating to said patient therapeutically effective amounts of the combination according to the invention.

Cabazitaxel and cisplatin may be administered simultaneously, semi-simultaneously, separately, or spaced out over a period of time so as to obtain the maximum efficacy of the combination; it may be possible for each administration to vary in its duration from a rapid administration to a continuous perfusion.

As a result, for the purposes of the present invention, the combination is not exclusively limited to the one which is obtained by physical association of the constituents, but also to those which permit a separate administration, which can be simultaneous or spaced out over a period of time.

In the combinations according to the invention, the application of the constituents of which may be simultaneous, separate or spaced out over a period of time, it is especially advantageous for the amount of cabazitaxel to represent from 10 to 90% by weight of the combination, it being possible for this content to vary in accordance with the nature of the associated substance, the efficacy sought and the nature of the cancer to be treated.

In the combination according to the invention, cabazitaxel and cisplatin are preferably administered parentally, for example intravenously.

The invention also concerns a pharmaceutical kit which comprises:

(iii) a first galenic formulation comprising cabazitaxel in the form of a free base or of an addition salt with a pharmaceutical acceptable acid, or in the form of a hydrate or of a solvate;
(iv) a second galenic formulation comprising cisplatin;

both galenic formulations (i) et (ii) being intended to be independently administered, each administration with regard to the other one being simultaneous, separated or spread in the time.

The invention also concerns the above pharmaceutical kit for its use in the treatment of neoplastic diseases, more particularly in the treatment of cancers.

The invention also concerns the above pharmaceutical kit adapted for an administration of cabazitaxel by perfusion at a dose of 15 $mg/m^2$ and for an administration of cisplatin by perfusion at a dose of 75mg/m2. The invention also concerns the above pharmaceutical kit where the cycle of administration of the two antitumoral agents is repeated with an interval between two administrations of cabazitaxel of three weeks.

In another aspect the invention provides for an article of manufacture comprising:

a packaging material ; the above disclosed pharmaceutical combination comprising cabazitaxel and cisplatin, cabazitaxel being in the form of a free base or of an addition salt with a pharmaceutical acceptable acid, or in the form of a hydrate or of a solvate; and a label or package insert contained within said packaging material indicating that said pharmaceutical combination is administered to the patient at a recommended dose (RD) or
a Maximum Tolerated doe (MTD), and in a plurality of subsequent doses at a recommended dose (RD) or a Maximum Tolerated doe (MTD), separated in time from each other by three weeks.

The combination is administered repeatedly in a course of several cycles according to a protocol that depends on the nature and on the stage of the cancer to be treated and also on the patient to be treated (age, weight, previous

treatment(s), etc.).

Examples of cycles and doses are given in the example 2 below.

Example 1:

The improved efficacy of the combination according to the invention may be demonstrated by determination of the therapeutic synergy as illustrated in the following example.

In this example, the effectiveness of a cabazitaxel/cisplatin combination of the invention for tumor growth inhibition was demonstrated in vivo.

The selected tumor model was a murine tumor, colon adenocarcinoma C51, grafted in the syngenic strain of mice of origin of the tumor, BALB/C mice [T.H. Corbett et al., Cancer, 40: 2660-2680 (1977)].

Cabazitaxel was weighed for each treatment and dissolved in ethanol. Treatment solutions were prepared first by mixing 1 volume of ethanolic stock solution and 1 volume of polysorbate 80, then by adding 18 volumes of glucose 5% in water.

Cabazitaxel was administered intravenously on days 5, 12 (or 13) after tumor implantation. Cisplatin was formulated in NaCl 0.9%, pH 4.5. Cisplatin was administered intravenously on days 5, 12 (or 13) after tumor implantation, with a 15 min or a 24h interval to cabazitaxel.

The results of the experiment are reported in Table 1.

Tumor doubling time was 2.5 days.

The following end points have been used:

- Toxicity was declared at dosages inducing ≥ 20% body weight loss or ≥ 10 % drug death Tumor growth inhibition was determined on day 20 post tumor implantation when the median tumor size in the control group was 1352 mg.

The tumors of treatment (T) and control (C) groups were measured when the median of control group reached approximately 750 to 1400 mg. The median tumor weight of each group was determined.

- Antitumor efficacy was determined by calculating the T/C value in percent:

T/C (%) = Median tumor weight of the Treated/ control x 100

According to NCI standards, a T/C < 42 % is the minimal level to declare activity. A T/C < 10 % is considered to indicate high antitumor activity and is the level used by NCI to justify further development (Decision Network-2 level, DN-2).

- To better quantify the antitumor activity, another end point, the log cell kill, was used.:

  log10 cell kill = (T-C) / [3.32 x (tumor doubling time in days)] (T meaning the median time of the treated mice to reach 750 mg and C the median time (17.6 days) of the control mice to reach the same size). No antitumor activity was declared for log cell kill < 0.7, and the treatment was declared highly active for log cell kill ≥ 2.8

- Therapeutic Synergism: a combination has therapeutic synergism if it is more active than the best single agent of the study (by at least 1 log cell kill).

Toxicity for cabazitaxel alone was observed at a dose of 32.3 mg/kg/injection, with 6/6 drug-related deaths occurring from day 19 to day 24. The highest nontoxic dose (HNTD) for cabazitaxel, 20 mg/kg/inj (total injected dose = 40 mg/kg), was found to be active with a log cell kill of 1.8. The lowest doses of 12.4 and 7.7 mg/kg/inj remained active with 1.4 and 1.3 log cell kill, respectively.

Toxicity for cisplatin alone was observed at a dose of 7 mg/kg/injection, with 2/6 drug-related deaths occurring on days 8 and 23, a 24.6% body weight loss being also observed at nadir on day 17, i.e. above the 20% threshold. The HNTD for cisplatin, 4.3 mg/kg/inj (total injected dose = 8.6 mg/kg), was found to be active with a log cell kill of 2.7. The lowest dose 2.7 mg/kg/inj remained active with 1.8 log cell kill.

Using a 15 min interval between administrations of the 2 agents, the combination of cisplatin at 3.5 mg/kg/inj with cabazitaxel at 10 mg/kg/inj was declared toxic, with 19.5 % body weight loss at nadir on day 20. The dose of 2.8 mg/kg/inj of cisplatin combined with 8 mg/kg/inj of cabazitaxel was considered to be the HNTD. Remarkably, this dose was found highly active with 4.6 log cell kill, clearly far more active than the activity of each agent administered alone. Likewise, a greater antitumor activity was also observed at the dose below the HNTD (2.1 mg/kg/inj of cisplatin with 6 mg/kg/inj of cabazitaxel) with 3.6 log cell kill.

We can thus conclude that this combination shows a therapeutic synergy.

Using a 24 h interval between administrations of the 2 agents, two different order of administrations have been evaluated, cabazitaxel first on days 5 and 12, cisplatin being administered 24h later on days 6 and 13, or the reverse sequence with cisplatin being administered first.

- Cabazitaxel first: The combination of cabazitaxel at 13 mg/kg/inj with cisplatin at 4.6 mg/kg/inj was declared toxic, with 1/6 drug-related death, and 27.5 % body weight loss at nadir on day 21. The dose of cabazitaxel at 10 mg/kg/inj followed 24h later by cisplatin at 3.5 mg/kg/inj was considered to be the HNTD. This dose was found highly active with 4.6 log cell kill, being clearly far more active than the activity of each agent administered alone, as previously observed in the 15min interval combination. Of interest, a greater antitumor activity was also observed at the 2 doses below the HNTD (cabazitaxel/cisplatin combination at 8/2.8 and 6/2.1 mg/kg/inj), with 4.1 and 3.7 log cell kill, respectively.

We can thus conclude that using this sequence of administration of 24h interval with cabazitaxel being injected first, this combination shows also a therapeutic synergy.

- Cisplatin first: The combination of cisplatin at 4.6 mg/kg/inj with cabazitaxel at 13 mg/kg/inj was declared toxic, with 20.6 % body weight loss at nadir on day 19. The dose of cisplatin at 3.5 mg/kg/inj followed 24h later by cabazitaxel at 10 mg/kg/inj was considered to be the HNTD. As previously observed when cabazitaxel was administered first, this HNTD was found highly active with 4.4 log cell kill. A greater antitumor activity was also observed at the 2 doses below the HNTD (cisplatin/cabazitaxel combination at 2.8/8 and 2.1/6 mg/kg/inj), with 4.1 and 3.5 log cell kill, respectively.

We can thus conclude that using this sequence of administration of 24h interval with cisplatin being injected first, this combination shows also a therapeutic synergy.

In conclusion, cabazitaxel-cisplatin combination shows therapeutic synergy whatever the sequence of administration of the agents (at the HNTD: 4.4 to 4.6 log cell kill for the combination versus 1.8 log cell kill for cabazitaxel alone and 2.7 log cell kill for cisplatin alone). In addition, this therapeutic synergy was maintained at the dosages below the HNTD at one (15min apart) and 2 (24h apart) dose levels.

**Table I: Combination of cabazitaxel and cisplatin against colon adenocarcinoma C51 implanted in BALB/c female mice.**

| Agent by IV route Dose in mg/kg/inj (total dose) | | Schedule in days | Drug death | % Body weight change at nadir (day) | T/C in % on day 20 | T-C in days (750 mg) | $log_{10}$ cell kill gross | Comments |
|---|---|---|---|---|---|---|---|---|
| **Cabazitaxel** | **Cisplatin** | | | | | | | |
| 32.3 (64.6) | - | 5, 12 | 6/6 | - | - | - | - | Toxic (6/6 DD) |
| 20.0 (40.0) | - | | 0/6 | -14.6 (19) | 0 | 15.2 | 1.8 | HNTD active |
| 12.4 (24.8) | - | | 0/6 | -8.1 (9) | 3 | 11.7 | 1.4 | Active |
| 7.7 (1 5.4) | - | | 0/6 | -6.8 (9) | 5 | 11.0 | 1.3 | Active |
| - | 7.0 (14.0) | 5,12 | 2/6 | -24.6 (17) | - | - | - | Toxic (2/6 DD; -25% BWC) |
| - | 4.3 (8.6) | | 0/6 | -7.8 (15) | 0 | 22.1 | 2.7 | HNTD active |
| - | 2.7 (5.4) | | 0/6 | -3.7 (7) | 2 | 15.1 | 1.8 | Active |
| **15 min interval combination** | | | | | | | | |
| 10.0 (20.0) | 3.5 (7.0) | 5, 12 | 0/6 | -19.5 (20) | - | - | - | Toxic (-20% BWC) |
| 8.0 (16.0) | 2.8 (5.6) | | 0/6 | -9.3 (18) | 0 | 38.4 | 4.6 | HNTD highly active |
| 6.0 (12.0) | 2.1 (4.2) | | 0/6 | -7.1 (9) | 0 | 30.1 | 3.6 | Highly active |

(continued)

| | | | | | | | | |
|---|---|---|---|---|---|---|---|---|
| **15 min interval combination** | | | | | | | | |
| 3.0 (6.0) | 1.1 (2.2) | | 0/6 | -3.4 (7) | 4 | 12.2 | 1.5 | Active |
| **24h interval combination, cabazitaxel, first** | | | | | | | | |
| 13.0 (26.0) | 4.6 (9.2) | 5, 12 | 1/6 | -27.5 (21) | - | - | - | Toxic (-28% BWC) |
| 10.0 (20.0) | 3.5 (7.0) | | 0/6 | -17.9 (17) | 0 | 37.8 | 4.6 | HNTD highly active |
| 8.0(16.0) | 2.8(5.6) | | 0/6 | -17.2(21) | 0 | 34.1 | 4.1 | Highly active |
| 6.0 (12.0) | 2.1 (4.2) | | 0/6 | -11.1 (19) | 0 | 30.3 | 3.7 | Highly active |
| 3.0 (6.0) | 1.1 (2.2) | | 0/6 | -4.3 (15) | 4 | 11.3 | 1.4 | Active |
| **24h interval combination, cisplatin, first** | | | | | | | | |
| 13.0(26.0) | 4.6(9.2) | 5, 12 | 0/6 | -20.6(19) | - | - | - | Toxic (-21% BWC) |
| 10.0 (20.0) | 3.5 (7.0) | | 0/6 | -14.4 (17) | 0 | 36.2 | 4.4 | HNTD highly active |
| 8.0 (16.0) | 2.8 (5.6) | | 0/6 | -16.3 (19) | 0 | 34.3 | 4.1 | Highly active |
| 6.0 (12.0) | 2.1 (4.2) | | 0/6 | -7.0 (18) | 0 | 29.3 | 3.5 | Highly active |
| 3.0 (6.0) | 1.1 (2.2) | | 0/6 | -0.9 (7) | 6 | 10.1 | 1.2 | Active |

Tumor doubling time = 2.5 days. Median tumor size in the control group on day 20 = 1352 mg. Time for median control tumor to reach 750 mg = 17.6 days. Abbreviations used: BWC = body weight change, T/C = tumor growth inhibition, T-C = tumor growth delay, HNTD = highest nontoxic dose, IV = intravenous.

Example 2:

This example describes a clinical study evaluating the safety, tolerability, pharmacokinetics and efficacy of a cabazitaxel/cisplatin combination of the invention given every 3 weeks in patients with advanced solid cancer.

**TITLE**

A Dose-Escalation Study Of The Safety, Tolerability, And Pharmacokinetics Of Cabazitaxel In Combination With Cisplatin Administered Every 3 Weeks In Subjects With Advanced Solid Malignancies

**STUDY OBJECTIVE(S)**

Primary Objectives:

- Part 1:

  To determine the Dose Limiting Toxicities (DLTs) and Maximum Tolerated Dose (MTD) of Cabazitaxel administered as a 1-hour infusion in combination with cisplatin every 3 weeks in patients (pts) with advanced solid malignancies.

- Part 2:

  To determine the antitumor activity of Cabazitaxel in combination with cisplatin, as assessed by objective response rate (ORR) according to RECIST criteria.

Secondary Objectives:

- To assess the safety profile of the combination regimen of Cabazitaxel with cisplatin.
- To assess the pharmacokinetics (PK) of Cabazitaxel and of cisplatin, and to evaluate any PK drug-drug interaction between the compounds following this schedule of administration.

- To determine Time To Progression (TTP) and Duration of Response (DR), of the extended cohort of pts treated at the MTD in Part 2 of the study and the patients who received the MTD in Part 1 component.

**STUDY DESIGN**

The study is designed in Parts 1 and 2 as an open-label, single arm, dose-escalation, multicenter, study of Cabazitaxel in combination with cisplatin, to determine:

- Part 1: the DLT's and MTD based on safety,
- Part 2: the anti-tumor activity of the combination regimen at the MTD in an extended cohort of pts,

In Part 1, as shown in the table below, cohorts of 3 to 6 pts will be treated with Cabazitaxel and cisplatin at Day 1 every 3 weeks. The starting dose (Dose Level 0) will be 20 mg/m2 for Cabazitaxel and 75 mg/m2 for cisplatin every three weeks.

Dose Escalation Schedule:

| Dose levels | Cabazitaxel, mg/m2 | Cisplatin, mg/m2 |
|---|---|---|
| -1 | 15 | 75 |
| 0 | 20 | 75 |
| +1 | 25 | 75 |

For safety reasons, the actual dose of Cabazitaxel will be adjusted to a maximum BSA of 2.1 $m^2$.

The dose escalation criteria as described in the table below must be met at each dose level during cycle 1 in order to enroll and treat pts at the next dose level.

Cabazitaxel dose escalation decision rules:

| Patients with Cycle 1 DLT at a Given Dose Level | Dose Escalation Decision Rule |
|---|---|
| 0 of first 3 patients | Escalate dose level and enter at least 3 pts at the next dose level, if that dose is available. |
| 1 out of the first 3 patients | Enter up to 3 additional pts at this dose level.<br>If 0 of the 3 additional pts experience DLT, then proceed to the next higher dose level, if that dose available<br>If >1 additional pts experience DLT, the next lower dose level will be selected if available. |
| 2 of the first 3 patients | Decrease dose to the next dose level if available. |
| 2 out of 6 patients | Decrease dose to the next dose level if available.<br>If not available, the MTD has been reached. |

At each given dose level, there will be a one-week gap to evaluate toxicity, between the inclusion of the first pt and the next 2 pts. Before escalating to the next dose level, at least 3 pts should be evaluable for the criteria defining a DLT.

Dose-Limiting Toxicities (DLT) and Maximum Tolerated Dose (MTD):

To qualify for DLT, the clinical adverse event (AE) or laboratory abnormality should be drug-related as assessed by the investigator or sponsor.

The DLTs will be defined (according to NCI-CTCAE version 3 grading scale) during the first treatment cycle, as follows:

- Non-hematological toxicity grade 3 or 4 except:
  - Grade 3 fever without documented infection
  - Grade 3 nausea, vomiting, or diarrhea in the absence of effective maximal therapy
  - Grade 3 mucositis/stomatitis in the absence of effective symptomatic treatment

- Grade 3 fatigue
- Grade 3 anorexia
- Grade 3 AST/ALT (aspartate aminotransferase / alanine aminotransferase) elevation that returns to baseline prior to next treatment cycle
- Grade 3 HSR (acute hypersensitivity reaction) in the absence of required premedication
- Peripheral neuropathy grade 3 that returns to grade 1 or less at the initiation of next treatment cycle
- Any other life-threatening clinical drug related-toxicity

• Hematological toxicity defined as:

- Febrile neutropenia: fever (of unknown origin without clinically or microbiologically documented infection) ≥38.5°C with neutropenia grade 3 or 4
- Neutropenia grade 4 lasting >7 days
- Platelets /Thrombocytopenia grade 4

In case of the occurrence of one DLT at the first cycle, 3 additional pts will be included at the same dose level and the MAD (Maximal Administered Dose) will be reached at the dose level when at least 2 pts develop a DLT at the first cycle.

Prophylactic and therapeutic use of hematopoietic growth factors is not permitted during the first 3 weeks of study treatment unless a hematological DLT is encountered.

Maximal Tolerated Dose (MTD):

The MTD will be defined as the highest dose at which 0 or 1 of 3 or 6 pts respectively experience DLT during the first 3 weeks of combination of Cabazitaxel and cisplatin. If there is a dose decrease to Dose Level -1 of Cabazitaxel, the MTD will be established at this dose level.

Once the MTD of Cabazitaxel in combination with cisplatin has been established, the safety, PK and preliminary efficacy of this regimen will be evaluated in an additional 15 patients in the part 2 component of the study.

Patients will be monitored closely for toxicity. In addition to optimizing supportive care, chemotherapy doses may be adjusted after the first cycle of therapy and recovery to grade ≤1 or baseline. Intrapatient dose escalation is not permitted. For those patients who have had a DLT, further treatment with a lower dose is at the investigator's discretion.

Further cycles will not be evaluable for MAD or MTD. However, safety and efficacy data will continue to be collected.

All patients treated in parts 1 and 2 will continue to receive treatment until disease progression, unacceptable toxicities/adverse events, withdrawal of consent, or investigator's decision to withdraw, whichever comes first.

**STUDY POPULATION**

**Inclusion criteria:**

- Age ≥ 18 years
- Histologically or cytologically confirmed advanced solid malignancy that is metastatic or unresectable, and for which standard curative measures do not exist, but for which cisplatin based therapy is appropriate
- Eastern Cooperative Oncology Group (ECOG) performance status (PS) ≤ 1
- Presence of measurable disease (part 2 only)
- No treatment with cisplatin within 6 months prior to the study start
- No cancer therapy within 3 weeks prior to the study start
- No concurrent treatment in another clinical trial or with any other cancer therapy
- No continued toxic effects of prior cancer therapy
- Adequate white blood cells, platelets, haemoglobin, and liver and kidney function.

**Exclusion criteria**

**[0096]** - inability to follow study requirements and schedule
- treatment of cancer within 3 weeks of study, concurrent treatment in another clinical trial or with any other cancer therapy
- serious medical illness at same time of study and/or significantly abnormal lab reports

- lack of pregnancy contraception (women of childbearing potential), pregnancy, or breast feeding.
- prior significant hearing or kidney problems
- continued toxic effects of prior chemotherapy
- cancers that cannot be physically measured (Part 2 only)
- Inadequate organ function as defined by:

Absolute neutrophil count (ANC) <1500/mm3; Platelets <75 000/mm3; Hemoglobin <9.0 g/dL; Prothrombin time/ International normalized ratio (PT/INR) >1.5; Estimated creatinine clearance (CrCl) <60 mL/min, or serum Creatinine ≥1.0 x ULN;

| | |
|---|---|
| Total bilirubin | > normal limits (WNL) |
| Alkaline phosphatase (AP) | >5.0 x ULN |
| Serum aspartate aminotransferase (AST; serum glutamate-oxalate transferase [SGOT] and serum alanine aminotransferase (ALT; serum glutamate-pyruvate transferase) [SGPT] | If AP is ≤2.5 x ULN, and ALT/AST >2.5 x ULN.<br>If AP is >2.5- ≤5.0 x ULN, and ALT/AST >1.5xULN |

**INVESTIGATIONAL PRODUCT(S)**

Cabazitaxel

Cabazitaxel is supplied as a sterile, non-pyrogenic, non aqueous yellowish to brownish yellow, 60 mg/1.5 ml concentrate for solution for infusion. It is packaged in 15 ml single dose clear type I glass vial. The solution contains the following excipient: Polysorbate 80. Solvent: The solvent for Cabazitaxel is supplied as a 13 % m/m ethanol solution in water for injection. This solvent is supplied in a 15 ml single dose clear type I glass vial. The preparation of the Cabazitaxel infusion solution for administration requires first the preparation of a premix solution at 60 mg/6 ml (10 mg/ml) (nominal concentration). Each vial of Cabazitaxel must be diluted with the entire content of one solvent vial. Each Cabazitaxel vial and each solvent vial are overfilled to ensure that a 60 mg dose can be extracted after the preparation of the premix solution. The premix solution must then be diluted in an infusion vehicle (0.9% NaCl or Glucose 5%) to obtain the required dose for administration.

Cisplatin:

Cisplatin 75 mg/m2 will be administered in 500 ml NaCl 0.9% IV over 1 hour on Day 1, 1 hour before Cabazitaxel administration. Commercially available cisplatin will be utilized to prepare the IV infusion. Appropriate pre and post supportive medications providing hydration, antiemetic and dexamethasone will be administered.

**Route(s) of administration:**

Cabazitaxel and cisplatin : intravenous.

**Dose regimen:**

**Part 1:**

Cisplatin will be administered first followed by Cabazitaxel infusion. Both infusions should be administered through different infusion lines.

a) On day 1 of each cycle, cisplatin infusion at 75 mg/m2 will be administered in 500 ml NaCl 0.9% IV over 1 hour.
b) On day 1 of each cycle, patients will receive Cabazitaxel, administered by IV infusion over 1 hour, at the dose specified for each level. IV premedications including antihistamines should be administered by infusion 30 minutes before the administration of Cabazitaxel.

For those patients who have had a DLT, further treatment with a lower dose is at the investigator's discretion. Further cycles will not be evaluable for MAD or MTD. However, safety and efficacy data will continue to be collected.

**Part 2:**

Patients will receive both Cabazitaxel and cisplatin at day 1 of each cycle in the same manner as Part 1.
Cycles lengths in both part 1 and part 2, for this treatment combination are 3 weeks.

**Parts 1 and 2:**

New cycles of therapy may not begin until ANC (Absolute neutrophil count) ≥1500/mm3, platelet count ≥75 000 /mm3, serum creatinine ≤1.5, blood urea nitrogen ≤25 mg/dl, liver function tests are within range as indicated in exclusion criteria, and non-hematologic toxicities (except alopecia, asthenia, local reactions, and other toxicities that are uncomfortable but do not cause serious morbidity to patients) have recovered to grade ≤1 or baseline.
Dose modification may be required as detailed in the protocol. A maximum of 2-weeks treatment delay is allowed between treatment cycles. Patients should discontinue study treatment if treatment delay is more than 2 weeks.

**PRIMARY ENDPOINT(S) AND MAIN SECONDARY ENDPOINT(S)**

The primary endpoint of the study will be:

Part 1: DLTs at cycle 1 of the combination of Cabazitaxel and cisplatin.
Part 2: Anti-tumor activity based on RECIST criteria, including patients at the MTD who have continued from Part 1.

Efficacy will be determined using objective responses (CR ie Complete Response and PR ie Partial Response) as assessed by investigators according to RECIST criteria. Confirmation of objective responses will be performed by repeat tumor imaging (CT (Computed Tomography) scans, MRI (Magnetic Resonance Imaging)) 4-6 weeks after the first radiological documentation of response.
Main secondary endpoint evaluations will include:

1. TTP (parts 1 and 2)
2. DR (parts 1 and 2)
3. Safety profile of the combination in terms of AEs/ SAEs and laboratory parameters
4. PK of Cabazitaxel (parts 1 and 2) and cisplatin (part 1)

**ASSESSMENT SCHEDULE**

Safety Data:

Vital signs, medical history, physical examinations, ECOG PS, EKG, and laboratory safety tests (including complete blood counts, serum chemistries, and urinalysis) will be obtained prior to drugs administration and at designated intervals throughout the study. Treatment-emergent adverse events (TEAEs) will be collected during the study and up to 30 days after the end of study treatment. Any SAEs considered related to the study medication will be collected regardless of when they occur. AEs will be graded according to the National Cancer Institute Common Terminology Criteria for Adverse Events, Version 3.0 (NCI CTCAE v.3.0).

Efficacy Data:

In parts 1 and 2, antitumor activity will be assessed by computerized tomography (CT) or magnetic resonance imaging (MRI) of the chest, abdomen, pelvis, and/or other areas of tumor burden. These exams will be performed at baseline (screening), at the end of each even-numbered treatment cycle (ie, days 15-21 of cycles 2, 4, 6, ...), whenever disease progression is suspected, and at the end of treatment/withdrawal visit, using the same method for each assessment. For bone, the scan will be performed at baseline and whenever new or worsening bone symptoms occur, and at time a tumor assessment is done to confirm a response.

Patients who discontinue study treatment prior to disease progression will continue to have tumor assessments every 6 weeks until disease progression or start of another anticancer therapy.
PK Data:

Part 1: Cabazitaxel PK - Blood samples (2 ml each) for PK analysis will be collected from all patients at cycle 1 as detailed in the protocol. Cisplatin PK - Blood samples (5 ml each) for PK analysis will be collected from all patients at cycle 1 for quantification of free and bound platinum derivative in plasma at appropriate duration as detailed in the protocol.

Part 2: Cabazitaxel PK - Blood samples (2 ml each) for PK analysis will be collected from all patients at cycle 1 and at cycle 2 as detailed in the protocol.

**STATISTICAL CONSIDERATIONS**

**Part 1:**

Approximately 15 patients will be required to establish the MTD and tolerability of the combination with a planned number of 3 dose levels (3 to 6 patients per dose level). Analysis population: Treated population defined as all patients who took at least one part of a dose of Cabazitaxel or cisplatin.

The statistical analysis will be descriptive. The analyses variables are:

- DLT as primary analysis variable,
- TEAE, Labs.

The cut off date for Part 1 safety analysis: end of cycle 1 received by the last patient included in this part.

**Part 2:**

Approximately 15 additional patients will be accrued additionally to the patients who received the MTD in the part 1 component.

Analysis populations:

- Treated population: all patients who took at least one part of a dose of study medication Cabazitaxel.

- Per-protocol population: treated patients who meet the following requirements: a) with minimal 2 cycles of treatment or early withdrawal due to toxicity, progression or death within the same period; b) Have valid baseline and at least one post-baseline tumor assessment. The analyses variables are:

    Efficacy:
    • Objective overall response (PR or CR) as primary endpoint,
    • TTP
    • DR

    Safety:
    • TEAE, SAEs, Labs.

Statistical Methods:

A 95% exact confidence interval will be provided for objective response rate for treated and per-protocol population. Kaplan-Meier curves and life tables will be provided for TTP treated population and Duration of response (DR) among the patients who have partial or complete responses.

The cut off date for the part 2: when the last patient has completed 6 cycles of treatment or discontinued study treatment (for disease progression, unacceptable toxicity, withdrawal of consent, or investigator's decision to withdraw), whichever comes first.

**DURATION OF STUDY PERIOD (per subject)**

The study consists of

- A maximum of 21-day screening phase,
- Registration,
- Study drugs administration starts within 5-business days of registration, with 21-days study treatment cycles. Cycle

lengths may be extended up to maximum of 2 additional weeks in case of unresolved toxicity.

- All patients treated will continue to receive treatment until disease progression, unacceptable toxicity, withdrawal of consent, or investigator's decision to withdraw, whichever comes first.
- There will be a 30-day follow-up visit after the last dose of study medication. In parts 1 and 2, patients who discontinued study treatment prior to disease progression will continue to have tumor assessments every 6 weeks until disease progression or start of another anticancer therapy.
- Cut-off date for parts 1 and 2: when the last patient has completed 6 cycles of treatment or discontinued study treatment (for disease progression, unacceptable toxicity, withdrawal of consent, or investigator's decision to withdraw), whichever comes first, in the corresponding part. Patients still receiving treatment at the cut-off date may continue to receive treatment beyond the cut-off date at investigator's discretion if benefiting.

**RESULTS**

- A total of 25 patients were recruited and treated in the study (Part 1 + Part 2) at 2 dose levels (DL). Ten (10) patients were treated during the dose escalation phase (Part 1): 7 patients at DL 20/75 and 3 patients at DL 15/75. Fifteen (15) patients were recruited and treated in Part 2 at the MTD determined in Part 1 (DL 15/75). In total, 18 patients were treated at the MTD in the study.
- Disease progression was the most frequent reason for treatment discontinuation overall (12/25 patients, 48.0%) and in patients treated at the MTD (10/18 patients, 55.6%). A total of 9 patients (9/25 patients, 36.0%) discontinued study treatment due to adverse event (AE). This included 4 patients (4/18 patients, 22.0%) treated at the MTD.
- The demographics and baseline characteristics of the population treated at the MTD are the following. The median age was 56.5 [32-71] years. The ECOG status was 0 or 1. Patients with a large variety of cancers were recruited (no more than 2 patients had the same tumor type); lungs, pancreas and prostate cancer were each presented by 2 patients; all other cancer types were each presented by one patient. The median number of organs involved was 3.0 [1-6]. Main organs involved (≥ 20%) were lymph nodes, lungs, and liver. Prior anti cancer therapies were surgery in 55.6%, radiotherapy in 66.7%, and prior chemotherapy in 100% of patients. The most common laboratory abnormalities (all grades) at baseline were anemia (77.8%) and lymphopenia (50.0%) for hematology and increased alkaline phosphatase (50.0%), hypercholestremia (33.3%), and increased ASAT (27.8%) for biochemistry.

**SAFETY**

- Two (2) of 6 evaluable patients experienced a DLT at dose level 0 (DL 20/75): 1 grade 3 acute renal failure and 1 febrile neutropenia. None of the 3 patients treated at dose level -1 (DL 15/75) experienced a DLT. DL 15/75 was defined as the MTD for Part 2.
- At the MTD, 60 cycles were administered to 18 patients. The median number of cycles per patient was 3 [1-8]. Three (3) patients received more than 6 cycles of study treatment: 2 patients received 7 cycles and 1 patient received 8 cycles. The median relative dose intensity (RDI) was 0.95 [0.74-1.02] for cabazitaxel and 0.98 [0.71-1.01] for cisplatin. Five (5) of 15 patients (33.3%) had at least 1 dose reduction for either cabazitaxel or cisplatin, with 1/15 patients (6.7%) with at least 1 dose reduction for cabazitaxel and 4/15 patients (26.7%) with at least 1 dose reduction for cisplatin. The most frequent (in at least 2 patients) TEAE leading to dose reduction was an increase in blood creatinine (in 2 patients).
- At the MTD, the 5 most frequent all grades TEAEs were nausea (77.8%), vomiting (72.2%), decreased appetite (66.7%), fatigue (61.1 %), diarrhea, and anemia (44.4% each). Grade 3-4 TEAEs occurred in 77.8% of the patients. The most frequent (in more than 2 patients) were nausea, anemia (each in 22.2% of patients), fatigue, and neutrophil count decreased (each in 16.7% of patients).
- TEAEs in the renal and urinary disorders System Organ Class were reported in 3/18 (16.7%) patients at the MTD (all grades and grade ≥3). Two (2) patients experienced grade 3 renal failure acute and 1 patient experienced grade 3 renal tubular necrosis.
- A total of 9 patients discontinued the study treatment due to AE(s). This included 4 patients at the MTD who discontinued treatment due to blood creatinine increase (2 patients), blood urea increase (1 patient) and drug hypersensitivity (1 patient).
- Nine (9) patients died due to disease progression. Two (2) patients experienced fatal TEAE(s): 1 patient in the DL 20/75 group experienced grade 4 septic shock (considered possibly related to study treatment) and died within 30 days from last study treatment administration, and 1 patient at the MTD experienced an AE of grade 4 disease

progression and died during the follow-up period. One patient died during the follow-up period from an unknown cause.

- Overall, 16/25 patients (64.0%) experienced serious TEAEs (all grades). At the MTD, 11/18 patients (61.1%) experienced serious TEAEs (all grades and grade ≥3); the most frequent serious TEAEs (all grades and grade ≥3) were nausea, vomiting, neutropenia, acute renal failure, and decreased appetite, each reported in 2/18 patients. The other serious TEAEs were reported in single patients.
- Neutropenia was the most common grade ≥3 hematological abnormality (based on laboratory values) overall (21/25 patients, 84.0%) and at the MTD (14/18 patients, 77.8%).

**EFFICACY**

- At the MTD, there was no CR or PR in the per-protocol (n = 15) and all-treated (n = 18) efficacy populations (Part 1 and Part 2).
- Eleven (11) patients of the all-treated population had SD. These patients presented with the following primary tumor sites: pancreas (2 patients), prostate (2 patients), lungs (2 patients), pleura, endometrial, esophagus, ovaries, and ependymoma. Among these patients, 1 patient with prostate adenocarcinoma had unconfirmed PR at Cycle 6 and progressed at Cycle 8.

**PHARMACOKINETICS**

- Exposure to cabazitaxel (AUC) on Cycle 1 increased in a dose proportional manner between 15 and 20 $mg/m^2$ of cabazitaxel when administered in combination with cisplatin: a 1.33-fold increase in dose resulted in 1.25-fold increase in AUC.
- The PK of cabazitaxel did not appear to be modified by the infusion of cisplatin.
- The PK of cisplatin (total and free) in this study (cisplatin dose: 75 $mg/m^2$) was similar to that previously reported ($AUC_{last}$ = 45 820 ng.h/mL for total platinum and $AUC_{last}$ = 4170 ng.h/mL for free platinum), indicating that cabazitaxel did not appear to alter the PK of cisplatin following this schedule of administration.

**CONCLUSION**

- The MTD of cabazitaxel is 15 $mg/m^2$ on Day 1 when combined with cisplatin at the dose of 75 $mg/m^2$ on Day 1, administered every 3 weeks.
- The DLTs reported at the MAD (renal function impairment and febrile neutropenia) were expected and consistent with a taxane combined with platinum.
- The Part 2 of the study confirmed the MTD, with 15 patients treated and 2 patients with DLT at Cycle 1 (1 febrile neutropenia and 1 grade 3 hypersensitivity reaction despite appropriate premedication). The overall safety profile was expected for a taxane combined with platinum and was manageable.
- The PK of cabazitaxel did not appear to be modified by the infusion of cisplatin.

Cabazitaxel did not appear to alter the PK of cisplatin (total and free).

## Claims

1. Combination comprising cabazitaxel, which may be in the form of anhydrous base, a hydrate or a solvate, and cisplatin.

2. Combination according to claim 1, where this antitumoral combination is well tolerated, does not exacerbate the toxicity of each of the antitumoral agents and which allows the treatment of tumors either by stabilizing or by inducing a partial or a complete regression of the tumor.

3. Combination according to claim 1 or 2 were cabazitaxel is in the form of a solvate.

**4.** Combination according to claim 3 were the solvate is an acetone solvate.

**5.** Combination according to claim 4 were the acetonate solvate contains between 5% and 8% by weight of acetone.

**6.** Combination according to claim 1 or 2 comprising an effective quantity of cabazitaxel and an effective quantity of cisplatin.

**7.** Combination according to claim 1 or 2 adapted for an administration of cabazitaxel by perfusion at a dose from 15 to 25 $mg/m^2$.

**8.** Combination according claim 1 or 2 adapted for an administration of cisplatin by perfusion at a dose of 75mg/m2.

**9.** Combination according to claim 7 or 8 adapted for an administration of cabazitaxel by perfusion at a dose of 15 $mg/m^2$ and for an administration of cisplatin by perfusion at a dose of 75mg/m2.

**10.** Combination according to claim 9, where the cycle of administration of the two antitumoral agents is repeated with an interval between two administrations of cabazitaxel of three weeks.

**11.** Combination according to claim 1 or 2 where the antitumoral combination shows therapeutic synergy.

**12.** Combination according to claim 1 or 2 where cabazitaxel and cisplatin are administered simultaneously, semi-simultaneously, separately, or spaced out over a period of time.

**13.** Combination according to claim 1 or 2 where the amount of cabazitaxel represents from 10 to 90% by weight of the combination.

**14.** Combination according to claim 1 or 2 where cabazitaxel and cisplatin are both administered parentally.

**15.** Combination according to claim 14 where cabazitaxel and cisplatin are both administered intravenously.

**16.** Combination according to any one of claim 1 or 2 for its use as a medicament in the treatment of cancer.

**17.** Use of cabazitaxel and cisplatin for the preparation of a combination according to any one of claim 1 or 2.

**18.** A pharmaceutical composition containing the combination according to any one of claim 1 or 2.

**19.** A pharmaceutical composition according to claim 18 for its use as a medicament in the treatment of cancer.

**20.** A pharmaceutical kit, which comprises:

(i) a first galenic formulation comprising cabazitaxel in the form of a free base or of an addition salt with a pharmaceutical acceptable acid, or in the form of a hydrate or of a solvate;
(ii) a second galenic formulation comprising cisplatin ;
both galenic formulations (i) et (ii) being intended to be independently administered, each administration with regard to the other one being simultaneous, separated or spread in the time.

**21.** A pharmaceutical kit according to claim 20 for its use in the treatment of cancers.

**22.** A pharmaceutical kit according to claim 20 or 21, adapted for an administration of cabazitaxel by perfusion at a dose of 15 $mg/m^2$ and for an administration of cisplatin by perfusion at a dose of 75mg/m2.

**23.** A pharmaceutical kit according to claim 22, where the cycle of administration of the two antitumoral agents is repeated with an interval between two administrations of cabazitaxel of three weeks.

Europäisches Patentamt
European Patent Office
Office européen des brevets

# EUROPEAN SEARCH REPORT

Application Number
EP 12 30 5109

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br><br><br><br><br>Y | Anonymous: "A Study to Evaluate the Effects of Combining Cabazitaxel With Cisplatin Given Every 3 Weeks in Patients With Advanced Solid Cancer (NCT00925743)", Clinicaltrials.gov<br>,<br>28 May 2010 (2010-05-28), pages 1-2, XP002639748,<br>Retrieved from the Internet:<br>URL:http://clinicaltrials.gov/archive/NCT00925743/2010_05_28<br>[retrieved on 2011-05-31]<br>* the whole document *<br>----- | 1-23<br><br><br><br><br><br><br><br><br><br><br><br>1-23 | INV.<br>A61K31/337<br>A61K33/24<br>A61P35/00 |
| Y,D | WO 2010/128258 A1 (SANOFI AVENTIS [FR]; MAGHERINI EMMANUELLE [FR])<br>11 November 2010 (2010-11-11)<br>* page 2, line 20 - page 3, line 8 *<br>----- | 3-5 | |
| Y | AGARWAL N ET AL: "Cabazitaxel for the treatment of castration-resistant prostate cancer",<br>FUTURE ONCOLOGY,<br>vol. 7, no. 1, January 2011 (2011-01), pages 15-24, XP009144697,<br>FUTURE MEDICINE LTD. GBR<br>ISSN: 1479-6694, DOI: 10.2217/FON.10.168<br>* the whole document *<br>* page 22, column 1, last 5 lines - column 2, line 2 *<br>-----<br>-/-- | 1-23 | TECHNICAL FIELDS SEARCHED (IPC)<br>A61K<br>A61P |

The present search report has been drawn up for all claims

| Place of search | Date of completion of the search | Examiner |
|---|---|---|
| Munich | 15 May 2012 | Rodríguez-Palmero, M |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 30 5109

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CROWN JOHN ET AL: "The taxanes: An update", LANCET (NORTH AMERICAN EDITION), vol. 355, no. 9210, 1 April 2000 (2000-04-01), pages 1176-1178, XP002639750, ISSN: 0099-5355 * page 1176, column 2, paragraph 5 - page 1177, column 2 * ----- | 1-23 | |
| Y | CROWN JOHN ET AL: "Platinum-taxane combinations in metastatic breast cancer: An evolving role in the era of molecularly targeted therapy.", BREAST CANCER RESEARCH AND TREATMENT, vol. 79, no. Supplement 1, 2003, pages S11-S18, XP002639751, ISSN: 0167-6806 * abstract * ----- | 1-23 | |
| Y | MCGUIRE WILLIAM P 3RD: "Current status of taxane and platinum-based chemotherapy in ovarian cancer.", JOURNAL OF CLINICAL ONCOLOGY : OFFICIAL JOURNAL OF THE AMERICAN SOCIETY OF CLINICAL ONCOLOGY, vol. 21, no. 10 Suppl, 15 May 2003 (2003-05-15), pages 133S-135S, XP002639752, ISSN: 1527-7755 * abstract * * page 133s, column 1, paragraph 2 * ----- -/-- | 1-23 | TECHNICAL FIELDS SEARCHED (IPC) |

The present search report has been drawn up for all claims

| Place of search | Date of completion of the search | Examiner |
|---|---|---|
| Munich | 15 May 2012 | Rodríguez-Palmero, M |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 30 5109

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | RIGAS JAMES R: "Taxane-platinum combinations in advanced non-small cell lung cancer: a review.", THE ONCOLOGIST, vol. 9 Suppl 2, 2004, pages 16-23, XP002639753, ISSN: 1083-7159 * abstract * ----- | 1-23 | |
| A | BOUCHET B P ET AL: "Cabazitaxel, a new taxane with favorable properties.", DRUGS OF TODAY (BARCELONA, SPAIN : 1998), vol. 46, no. 10, October 2010 (2010-10), pages 735-742, XP002639754, ISSN: 1699-3993 * the whole document * ----- | 1-23 | |
| | | | TECHNICAL FIELDS SEARCHED (IPC) |

The present search report has been drawn up for all claims

1

| Place of search | Date of completion of the search | Examiner |
|---|---|---|
| Munich | 15 May 2012 | Rodríguez-Palmero, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 30 5109

This annex lists the patent family members relating to the patent documents cited in the above-mentioned European search report. The members are as contained in the European Patent Office EDP file on
The European Patent Office is in no way liable for these particulars which are merely given for the purpose of information.

15-05-2012

| Patent document cited in search report | Publication date | Patent family member(s) | Publication date |
|---|---|---|---|
| WO 2010128258 A1 | 11-11-2010 | AU 2010244253 A1 | 24-11-2011 |
| | | CA 2761079 A1 | 11-11-2010 |
| | | CO 6390103 A2 | 29-02-2012 |
| | | DO P2011000336 A | 15-12-2011 |
| | | EP 2427187 A1 | 14-03-2012 |
| | | FR 2945211 A1 | 12-11-2010 |
| | | FR 2945212 A1 | 12-11-2010 |
| | | KR 20120008069 A | 25-01-2012 |
| | | SG 175894 A1 | 29-12-2011 |
| | | US 2012115806 A1 | 10-05-2012 |
| | | WO 2010128258 A1 | 11-11-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9630355 A
- WO 2010128258 A
- EP 0817779 B1
- US 5847170 A
- WO 200502846 A


### Non-patent literature cited in the description


- *J. Pharm. Sci.,* vol. 64 (8), 1269-1288
- Pharmaceutical salts. *J.Pharm.Sci.,* 1977, vol. 66, 1-19
- **T.H. CORBETT et al.** *Cancer,* 1977, vol. 40, 2660-2680
- Cancer Drug Development, Part B. **F.M. SCHABEL et al.** Methods in Cancer Research. Academic Press Inc, 1979, vol. 17, 3-51